# FASCICULE DE BREVET EUROPEEN

(11) **EP 1 634 582 B1**
(45) Date de publication et mention de la délivrance du brevet: **02.03.2011**
(21) Numéro de dépôt: 05291314.2
(22) Date de dépôt: 20.06.2005
(51) Int. Cl.: A61K 8/86, A61K 8/365, A61Q 5/02, A61Q 19/10

(54) **Composition de nettoyage moussante**
Schäumendes Reinigungsmittel
Foaming cleansing composition

(30) Priorité: 26.07.2004 FR 0451653
(43) Date de publication de la demande: 15.03.2006
(73) Titulaire: L'Oréal, 75008 Paris (FR)
(72) Inventeur: Aubrun-Sonneville, Odile, 92160 Antony (FR); Sebillotte-Arnaud, Laurence, 94240 L'Hay les Roses (FR)
(74) Mandataire: Duvert, Sandra

(56) Documents cités:
- EP-A- 1 025 833
- EP-A- 1 029 535
- US-A- 5 977 036
- US-A- 6 010 990
- PATENT ABSTRACTS OF JAPAN vol. 017, no. 342 (C-1076), 29 juin 1993 (1993-06-29) & JP 05 043434 A (KAO CORP), 23 février 1993 (1993-02-23)

## Description

L'invention a pour objet une composition de nettoyage moussante, rinçable à l'eau, et ayant l'aspect d'un gel transparent, comprenant au moins un tensioactif ionique carboxylique et au moins un composé oxyéthyléné, ainsi qu'à ses utilisations dans les domaines cosmétique ou dermatologique, notamment comme produit de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Pour le nettoyage de la peau, il est connu d'utiliser des gels aqueux détergents moussants. Leur action nettoyante est apportée par les tensioactifs qu'ils contiennent, ces tensioactifs mettant en suspension les résidus gras et les pigments des produits de maquillage. Ces gels sont efficaces et agréables à utiliser du fait qu'ils moussent. On cherche notamment à faire des gels nettoyants moussants transparents car, tout comme l'eau, la transparence est le symbole de pureté et donc de propreté, et les gels transparents sont ainsi particulièrement appréciés des utilisateurs. Les gels transparents moussants destinés au nettoyage du visage ou du corps génèrent très souvent des mousses aérées et légères. Toutefois, après rinçage, la peau est souvent glissante et il ne reste pas la sensation d'une peau propre du fait de la présence d'un résidu filmogène sur la peau, difficile à éliminer. Une façon d'obtenir des mousses denses, à fines bulles et se rinçant rapidement sans laisser de film consiste à utiliser des savons (sels d'acides gras) comme tensioactifs principaux. Toutefois, les compositions avec savon sont moins bien tolérées, particulièrement par les peaux sensibles car elles donnent une sensation de sécheresse et peuvent être irritantes. En outre, elles ne sont pas transparentes mais le plus souvent opaques en raison de la faible solubilité des savons.

Il subsiste donc le besoin d'un gel transparent moussant ne comportant pas de savon et ayant une bonne qualité de mousse tout en ayant une bonne qualité de rinçage et une bonne tolérance oculaire et cutanée.

La demanderesse a découvert de manière surprenante qu'on pouvait atteindre le but de l'invention et obtenir un gel transparent moussant ayant à la fois de bonnes propriétés cosmétiques (qualités de la mousse et qualité du rinçage) et de bonnes propriétés de tolérance, en utilisant comme tensioactif, au moins un alkylglycol carboxylate, et en y associant un composé oxyéthyléné ayant un poids moléculaire supérieur à 300.000 g/mole.

Certes, il est connu dans l'art antérieur, des compositions moussantes contenant des composés oxyéthylénés. Ainsi, le document JP-A-05/043434 décrit des shampooings contenant des tensioactifs de type alkyl saccharides et des polyéthylène glycols de poids moléculaire supérieur à 500.000 g/mole. Toutefois, l'association décrite conduit à des compositions qui ne sont pas homogènes mais se séparent en deux phases.

Le document JP-A-03/174497 décrit des compositions liquides détergentes contenant un ester gras de fructose et un polyéthylène glycol de poids moléculaire compris entre 194 g/mole et 10.000 g/mole. Toutefois, les propriétés moussantes d'une telle composition s'avèrent insuffisantes.

La composition selon l'invention présente l'avantage d'être stable, non irritante et d'avoir de bonnes performances de mousse.

Ainsi, la présente demande a pour objet une composition de nettoyage pour application topique, contenant, dans un milieu aqueux physiologiquement acceptable, au moins un tensioactif anionique choisi parmi les acides alkyl glycol carboxyliques et leurs sels, et au moins un composé oxyéthyléné ayant un poids moléculaire égal ou supérieur à 300.000 g/mole.

On entend ici par « application topique » une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable. Par ailleurs, on entend par « milieu physiologiquement acceptable », un milieu compatible avec la peau, les lèvres, le cuir chevelu, les cils, les yeux, les ongles et/ou les cheveux. La composition peut constituer notamment une composition cosmétique ou dermatologique.

Par ailleurs, on entend par « milieu aqueux », un milieu comportant une quantité d'eau d'au moins 35 % en poids, allant de préférence de 35 à 98 % en poids et mieux de 40 à 80 % en poids par rapport au poids total de la composition. Le milieu aqueux des compositions moussantes de l'invention peut contenir, outre l'eau, un ou plusieurs solvants choisis parmi les alcools inférieurs comportant de 1 à 6 atomes de carbone, tels que l'éthanol; les polyols tels que la glycérine, les glycols comme le butylène glycol, l'isoprène glycol, le propylène glycol et les polyéthylène glycols de poids moléculaire inférieur à 10.000 tels que le PEG-8, le sorbitol, les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

Les compositions de l'invention constituent des compositions de nettoyage moussantes et rinçables, utilisables dans le domaine du nettoyage de la peau, des cheveux ou des muqueuses.

La composition obtenue se présente sous forme d'un gel transparent. Le mot « transparent » signifie que la composition a une turbidité inférieure ou égale à 500 NTU. Les NTU (Nephelometric Turbidity Units) sont les unités de mesure de la turbidité d'une composition. La mesure de turbidité peut être faite par exemple avec un turbidimètre model 2100P de la société HACH Compagny, les tubes utilisés pour la mesure étant référencés AR397A cat 24347-06. Les mesures sont effectuées à température ambiante (20°C à 25°C). La transparence d'une composition peut se mesurer soit par le coefficient de transmittance à 600 nm soit par la turbidité. La composition de l'invention a un coefficient de transmittance à 600 nm allant de 10 à 90 % ou bien une turbidité allant de 2 à 500 NTU, et de préférence de 5 à 300 NTU.

Par ailleurs, la viscosité des compositions selon l'invention va de préférence de 0,01 à 50 Pa.s, mesurée à 25°C à l'aide du Rheomat RM180 de Rheometric Scientific à 200 rpm (tours par minute), mesurée 10 minutes après la mise en rotation du mobile. L'appareil est équipé d'un mobile différent selon les viscosités, par exemple d'un mobile 2 pour les gammes de viscosités inférieures à 0,7 Pa.s, d'un mobile 3 pour les gammes de viscosités de 0,2 à 4 Pa.s, et d'un mobile 4 pour les viscosités supérieures à 2.

Les compositions de l'invention ont l'avantage d'être très stables et de ne présenter ni déphasage ni phénomène de recristallisation au stockage de 4°C à 45°C.

### Tensioactif anionique

La composition selon l'invention comprend au moins un tensioactif anionique choisi parmi les acides alkyl glycol carboxyliques (ou acides 2-(2-Hydroxyalkyloxy acétique), et leurs sels.

Ces tensioactifs peuvent notamment avoir la formule (I) suivante :

R₁-CHOH-CH₂-O-CH₂-COO⁻ X⁺ (I)

dans laquelle R₁ désigne un radical alkyle saturé ou insaturé, linéaire ou ramifié, comportant de 8 à 30 atomes de carbone, et X désigne l'hydrogène ou un cation minéral ou organique, tel que ceux issus d'un métal alcalin (par exemple Na+, K+), NH4+, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Les acides hydroxy-2 alkyl carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Parmi les tensioactifs de formule (I), on peut citer notamment le lauryl glycol carboxylate de sodium, commercialisé sous les dénominations BEAULIGHT SHAA® ou BEAULIGHT LCA-25N® par la société SANYO, ou sa forme acide correspondante commercialisée sous la dénomination BEAULIGHT SHAA (Acid Form)® par la société SANYO.

La quantité de tensioactifs anioniques de type alkyl glycol carboxylique peut aller par exemple de 0,5 à 20 % en poids (en matière active), de préférence de 1 à 17 % en poids par rapport au poids total de la composition finale.

### Tensioactifs additionnels

La composition selon l'invention peut comprendre, outre le tensioactif anionique décrit ci-dessus, un ou plusieurs tensioactifs additionnels choisis parmi les tensioactifs non ioniques, amphotères et anioniques, et leurs mélanges.

La quantité totale de tensioactifs (en matière active) peut aller par exemple de 2 à 50 % en poids, de préférence de 3 à 20 % en poids par rapport au poids total de la composition. Le rapport en poids (en matière active) du tensioactif de type alkyl glycol carboxylique par rapport aux autres tensioactifs va de préférence de 100/0 à 10/90 et mieux de 90/10 à 25/75.

### - Tensioactifs non ioniques

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la société Seppic ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

Selon un mode préféré de réalisation de l'invention, la composition contient comme tensioactif additionnel, un alkylpolyglucoside.

### - Tensioactifs amphotères

Les tensioactifs amphotères et zwitterioniques peuvent être choisis par exemple parmi les dérivés bétaïnes, amphoacétates, hydroxylsultaines, et leurs mélanges.

Comme bétaïnes, on peut citer par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyéthylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom CTFA : sodium cocamphoacetate).

Comme hydroxylsultaïnes, on peut citer la cocamidopropyl hydroxysultaine comme le produit commercialisé sous la dénomination REWOTERIC AM CAS par la société Golschmidt-Degussa.

### - Tensioactifs anioniques :

On peut utiliser par exemple comme tensioactifs anioniques, les carboxylates, les alkylsulfates, les sulfonates, les sulfosuccinates, les alkylsulfoacétates, les phosphates, les polypeptides, les dérivés anioniques d'alkylpolyglucosides, et leurs mélanges.

Comme carboxylates, on peut citer :
- Les amido éthercarboxylates (AEC), comme le Lauryl amido ether carboxylate de sodium (3 OE), commercialisé sous la dénomination AKYPO FOAM 30® par la société Kao Chemicals.
- les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX ® par la société Nikkol.
- les sels alcalins de N-acylaminoacides,
- les sarcosinates, comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN ® par la société Nikkol.
- les alaninates, comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA ® par la société Kawaken.
- Les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
- les aspartates, comme le mélange de N-lauroyl aspartate de triethanolamine /N-myristoyl aspartate de triethanolamine commercialisé sous la dénomination ASPARACK ® par la société Mitsubishi.
- Les glycinates, comme le N-cocoyl glycinate de sodium commercialisé sous les dénominations AMILITE GCS-12® et AMILITE GCK 12 par la société Ajinomoto.
- Les citrates tels que le mono-ester citrique d'alcools de coco oxyéthylénés (9 moles), commercialisé sous la dénomination WITCONOL EC 1129 par la société Goldschmidt.
- les galacturonates tels que le dodeécyl d-galactoside uronate de sodium commercialisé par la société Soliance.
- les sels d'acides gras (savons) ayant une chaîne alkyl en C6 à C22, neutralisés par une base organique ou minérale telle que la potasse, la soude, la triéthanolamine,la N-methyl glucamine, la lysine et l'arginine.

Comme alkyl sulfates oxyéthylénés ou non, ton peut citer par exemple le Lauryl éther sulfate de sodium (C12-C14 70/30) (2,2 OE) commercialisé sous la dénomination SIPON AOS 225® par la société Cognis, le Lauryl éther sulfate d'ammonium (C12-C14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le m2lange de lauryl- et oleyl- éther sulfate de sodium et magnésium, commercialisé sous la dénomination EMPICOL BSD 52 par la société Albright & Wilson.

Comme sulfonates, on peut citer par exemple :
- Les alpha oléfines sulfonates tels que l'alpha oléfine sulfonate de sodium (C14-16) commercialisé sous les dénominations BIO-TERGE AS-40® et BIO-TERGE AS-40 CG® par la société Stepan, ou commercialisé sous la dénomination WITCONATE AOS PROTEGE®, SULFRAMINE AOS PH 12® par la société Witco, l'oléfine sulfonate de sodium secondaire commercialisé sous la dénomination HOSTAPUR SAS 30® par la société Clariant.
- Les iséthionates tels que le Cocoyl isethionate de sodium commercialisé sous la dénomination JORDAPON Cl P® par la société JORDAN.
- Les taurates tels que le sel de sodium du methyltaurate d'huile de palmiste commercialisé sous la dénomination HOSTAPON CT PATE® par la société Clariant, le N-cocoyl N- methyltaurate de sodium commercialisé sous la dénomination HOSTAPON LT-SF® par la société Clariant ou NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyethylene (3 OE) commercialisé sous la dénomination SETACIN 103 SPECIAL® par la société Zschimmer Schwarz,, commercialisé sous la dénomination REWOPOL SB-FA 30 K 4® par la société Witco, le sel disodique d'un hemi-sulfosuccinate des alcools C12-C14 commercialisé sous la dénomination SETACIN F SPECIAL PASTE® par la société Zschimmer Schwarz, l'oleamido sulfosuccinate di-sodique oxyéthyléné (2 OE) commercialisé sous la dénomination STANDAPOL SH 135® par la société Cognis, le monosulfosuccinate d'amide laurique oxyéthyléné (5 OE) commercialisé sous la dénomination LEBON A-5000® par la société SANYO.

Comme alkyl sulfoacétates, on peut citer par exemple le mélange de lauryl sulfoacetate de sodium, et le lauryl ether sulfosuccinate di-sodique, commercialisé sous la dénomination STEPAN-MILD LSB par la société Stepan.

Comme phosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodecyl-phosphorique, le mélange mono-di-ester (majoritaire) commercialisé sous la dénomination CRAFOL AP-31® par la société Cognis, l'acide octyl-phosphorique, le mélange mono-di-ester commercialisé sous la dénomination CRAFOL AP-20® par la société Cognis, le mélange de mono et de di ester phophorique de 2-butyl octanoéthoxylé (7 MOLES D'OE) commercialisé sous la dénomination ISOFOL 12 7 EO-PHOSPHATE ESTER® par la société Condea.

Comme polypeptides, on peut citer par exemple ceux obtenus par condensation d'une chaîne grasse sur les aminoacides du blé et de l'avoine, tels que le sel de potassium de la lauroyl protéine de blé hydrolysée commercialisé sous la dénomination AMINOFOAM W OR® par la société Croda, la Cocoyl protéine de soja hydrolysée, le sel de tri-éthanolamine commercialisé sous la dénomination MAY-TEIN SY® par la société Maybrook, le sel de sodium de lauroyl amino acides d'avoine commercialisé sous la dénomination PROTEOL OAT® par la société Seppic, l'hydrolysat de collagène greffé sur acide gras de coprah commercialisé sous la dénomination GELIDERM 3000® par la société Deutsche Gelatine, les protéines de soja acylées par des acides de coprah hydrogénés, commercialisées sous la dénomination PROTEOL VS 22® par la société Seppic.

Comme dérivés anioniques d'alkyl polyglucoside, on peut citer les citrates, tartrates, sulfosuccinates, carbonates et éthers de glycérol fabriqués à partir des alkyl polyglucosides, comme le sel de sodium d'ester tartrique de cocoyl polyglucoside (1.4) commercialisé sous la dénomination EUCAROL AGE-ET® par la société Cesalpinia, le sel di-sodique d'ester sulfosuccinique de cocoyl polyglucoside (1.4) commercialisé sous la dénomination ESSAI 512 MP® par la société Seppic, le sel de sodium d'ester citrique de cocoyl polyglucoside (1.4) (1.4) commercialisé sous la dénomination EUCAROL AGE-EC® par la société Cesalpinia, le lauryl polyglucoside éther carboxylate de sodium commercialisé sous la dénomination PLANTAPON LGC SORB par la société Cognis.

Selon un mode préféré de réalisation de l'invention, la composition contient comme tensioactif additionnel, un tensioactif non ionique choisi parmi les alkylpolyglucosides.

### Composés oxyéthylénés

Les composés oxyéthylénés pouvant être utilisés dans la composition de l'invention sont ceux ayant un poids moléculaire supérieur à 300.000, le poids moléculaire allant de préférence de 400.000 à 4.10⁶ et mieux de 500.000 à 2.10⁶.

Selon un mode préféré de réalisation de l'invention, le composé oxyéthyléné est un composé de formule (II) :

H(OCH₂CH₂)ₙOH (II)

où n est un nombre entier allant de 7000 à 90.000.

Comme composé oxyéthyléné utilisé de préférence dans la composition de l'invention, on peut citer notamment le PEG 14M (formule (II) où n est 14000) tel que le produit commercialisé sous la dénomination Polyox WSR 205 par la société Amerchol, le PEG-45M (formule (II) où n est 45000) tel que le produit commercialisé sous la dénomination Polyox WSR N-60 K par la société Amerchol, et leurs mélanges.

Le composé oxyéthyléné est présent dans la composition de l'invention en une quantité allant de préférence de 0,1 à 5 % en poids et mieux de 0,2 à 3 % en poids par rapport au poids total de la composition.

### Additifs

La composition de l'invention peut contenir tous les additifs ou actifs classiquement utilisés dans les produits de nettoyage. On peut citer par exemple les conservateurs ; les séquestrants (EDTA) ; les antioxydants ; les parfums ; les matières colorantes comme les colorants solubles et les pigments ; les nacres ; les charges minérales ou organiques, apportant de la viscosité, matifiantes, blanchissantes ou exfoliantes ; les filtres solaires ; les actifs cosmétiques ou dermatologiques hydrophiles ou lipophiles, tels que les vitamines hydrosolubles ou liposolubles, les antiseptiques, les antiséborrhéïques, les antimicrobiens tels que le peroxyde de benzoyle, l'acide salicylique, le triclosan, l'acide azélaïque, la niacinamide (vitamine PP), et aussi les azurants optiques ; les corps gras incompatibles avec le milieu aqueux, comme les huiles ou les cires ; les agents régulateurs de viscosité ou épaississants, ou d'autres agents ayant pour effet d'améliorer les propriétés cosmétiques de cheveux ou de la peau, tels que les polymères anioniques, non ioniques, cationiques ou amphotères. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

La composition de l'invention peut notamment contenir des agents épaississants dont la quantité dépend de la viscosité souhaitée pour la composition finale. Les agents épaississants peuvent être présents à des concentrations allant de préférence de 0,05 à 10 % en poids et de préférence allant de 0.05 à 5 % en poids par rapport au poids total de la composition.

L'agent épaississant peut être choisi notamment parmi les polymères épaississants, les particules, les électrolytes (sels), les amides grasses, et leurs mélanges.

Les polymères épaississants peuvent être anioniques, amphotères, cationiques ou non ioniques, réticulés ou non, modifiés hydrophobes ou non, naturels ou de synthèse.

Comme polymères épaississants, on peut utiliser par exemple des polymères dérivés d'acide carboxylique, de polyacrylamide et/ou d'acide acrylamido-2-méthyl propane sulfonique. Le ou les polymères dérivés carboxyliques peuvent des polymères associatifs (c'est-à-dire comportant un groupement hydrophobe) ou non associatifs, solubles ou dispersibles dans l'eau, gonflants en milieu alcalin ou non. Ils peuvent être sous forme de poudre, de latex, en émulsion ou dispersés dans l'eau. Les polymères peuvent être non ioniques, anioniques, cationiques, zwitterioniques ou amphotères. Les monomères présents dans les polymères sont choisis de préférence parmi les monomères styrène, butadiène, éthylène, acrylonitrile, chloroprène, chlorure de vinylidène, isoprène, isobutylène, chlorure de vinyle, et esters d'acides acrylique, methacrylique, vinylacétique, maléique, crotonique et itaconique. Ces monomères peuvent être utilisés seuls ou en combinaison ou peuvent être mélangés avec un ou plusieurs monomères ioniques comme par exemple les acides acryliques ou methacryliques sous forme chargée.

Les polymères anioniques préférés contiennent un monomère dérivé d'acide acrylique ou méthacrylique et sont partiellement neutralisés comme par exemple les polymères commercialisés sous les dénominations Carbopol 981 et Carbopol 1382 par la société Noveon, et le polymère commercialisé sous la dénomination Acrysol 22 par la société Röhm & Haas.

Comme polymères épaississants non ioniques, on peut utiliser des dérivés oxyalkylénés d'esters d'acide gras ou d'éthers d'alcools gras, ou des polysaccharides. On peut citer comme dérivés oxyalkylénés d'esters d'acide gras ou d'éthers d'alcools gras, notamment les dérivés alkyl ou acyl éthoxylés de polyols, qui peuvent être en particulier des dérivés oxyéthylénés d'esters d'acide gras en C6-C30 ou d'éthers d'alcool gras en C6-C30, et de polyols tels que glycérol, sorbitol, glucose, pentaerythritol, ces dérivés oxyéthylénés comportant généralement de 50 à 500 groupes oxyéthylénés et de préférence de 100 à 300 groupes oxyéthylénés. Comme composés de ce type, on peut citer par exemple le stéarate de glycéryle oxyéthyléné (200 OE) tel que le produit commercialisé sous la dénomination SIMULSOL 220 TM ® par la société Seppic, le tétrastéarate de pentaérythrityle oxyéthyléné (150 OE) tel que le produit commercialisé sous la dénomination CROTHIX ® par la société Croda, le di-oléate de méthylglucose oxyéthyléné (120 OE) tel que le produit commercialisé sous la dénomination GLUCAMATE DOE-120 VEGETAL® par la société Amerchol, le triisostéarate de sorbitan oxyéthyléné (160 OE) tel que le produit commercialisé sous la dénomination RHEODOL TW IS399C par la société Kao Chemicals, et leurs mélanges. Comme polysaccharides, on peut citer la gomme de xanthane et des gommes analogues, et les dérivés de cellulose comme le Cetyl hydroxyethylcellulose commercialisé sous la dénomination Natrosol plus grade 330 CS par la société Hercules, et leurs mélanges.

Comme particules épaississant les formules, on peut utiliser les argiles comme les hectorites telles que la Bentone MA commercialisée par la société Elementis Specialities.

Comme amides grasses, on peut utiliser par exemple la cocamide MEA, la cocamide MIPA, et leurs mélanges.

Comme électrolytes, on peut utiliser par exemple le chlorure de sodium, le chlorure de potassium, le chlorure de magnésium, et des sels analogues, et leurs mélanges.

Les compositions selon l'invention sont stables, donnent une mousse fine et ont une très bonne rinçabilité. Elles peuvent constituer par exemple un produit de nettoyage et/ou de démaquillage de la peau, du cuir chevelu et/ou des cheveux, un produit de gommage et/ou un produit exfoliant pour la peau. Elles peuvent constituer plus particulièrement une composition de nettoyage de la peau.

Un autre objet de l'invention consiste en l'utilisation cosmétique de la composition telle que définie ci-dessus, comme produit de nettoyage et/ou de démaquillage de la peau, du cuir chevelu et/ou des cheveux, et/ou comme produit de gommage et/ou produit exfoliant pour la peau.

Les compositions selon l'invention peuvent être utilisées de deux façons :
- la première utilisation consiste à étaler le gel dans les mains, à l'appliquer sur le visage ou sur le corps puis à le masser en présence d'eau pour développer la mousse directement sur le visage ou le corps.
- l'autre utilisation possible de ce type de produit consiste à développer la mousse dans les paumes des mains avant de l'appliquer sur le visage ou le corps.

Dans les deux cas, la mousse est ensuite rincée.

Les compositions selon l'invention peuvent constituer aussi une composition pour le traitement des peaux grasses, notamment quand elles contiennent un actif spécifique de traitement des peaux grasses tels que, par exemple, l'acide salicylique, l'acide azélaïque, le triclosan, le piroctone olamine, la niacinamide (vitamine PP).

Un autre objet de l'invention est l'utilisation de la composition telle que définie ci-dessus pour la préparation d'une composition destinée à traiter la peau grasse.

Les exemples qui suivent servent à illustrer l'invention sans toutefois présenter un caractère limitatif. Les quantités indiquées sont en % en poids sauf mention contraire.

Dans le tableau des exemples, tous les pourcentages sont exprimés en poids de matière active (M.A.)

### Performances sensorielles :

Pour certains des exemples, les performances sensorielles des compositions (qualités de mousse) ont été déterminées selon le protocole décrit ci-dessous.

Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- travailler le produit à nouveau pendant 10 secondes,
6- ajouter 2 ml d'eau et travailler le produit à nouveau pendant 10 secondes,
7- rincer les mains sous l'eau ,
8- les essuyer.

Les critères sont évalués à chaque étape du protocole suivi, et ils sont notés sur une échelle de 0 à 10.
- étapes 4-5 : évaluation de la qualité de mousse (étape 6)
- *Le volume de mousse :* la note attribuée est d'autant plus élevée que le volume est grand.
- *La taille des bulles composant la mousse :* la note attribuée est d'autant plus élevée que les bulles sont grosses.
- *La tenue de la mousse :* la note attribuée est d'autant plus élevée que la mousse est élastique et ne coule pas
- étape 7 : évaluation pendant le rinçage
- *Le rinçage :* la note attribuée est d'autant plus faible que la présence d'un film glissant difficile à éliminer est grande, ce qui signifie que la note est d'autant plus forte que le produit se rince facilement sans laisser de film.

Le panel d'évaluation est constitué de 4 experts entraînés. La moyenne des quatre notes permet de comparer les compositions selon chacun des critères.

### Exemples 1 et 2 selon l'invention et exemples comparatifs 1 à 3

| Composition | Exemple 1 selon l'invention | Exemple 2 selon l'invention | Exemple comparatif 1 | Exemple comparatif 2 | Exemple comparatif 3 |
|---|---|---|---|---|---|
| Sodium Lauryl Glycol Carboxylate (1) | 7,8 | 7,8 | 7,8 | 7,8 | 7,8 |
| Coco-glucoside (2) | 6,5 | 6,5 | 6,5 | 6,5 | 6,5 |
| PEG-5M (P.M.=220018) (3) | 0 | 0 | 0 | 1 | 0 |
| PEG-14M (P.M.=616018) (4) | 1 | 0 | 0 | 0 | 0 |
| PEG-45M (P.M.=1.980.018) (5) | 0 | 1 | 0 | 0 | 0 |
| PEG-180 (P.M. = 7938) (6) | 0 | 0 | 1 | 0 | 0 |
| Acide citrique | 0,18 | 0,3 | 0,13 | 0,3 | 0,10 |
| DDM Hydantoin | 0 | 0,2 | 0,2 | 0,2 | 0,2 |
| Sodium Méthylparaben | 0 | 0,3 | 0,3 | 0,3 | 0,3 |
| Imidazolidinyl urée | 0,2 | 0 | 0 | 0 | 0 |
| Eau | Qsp 100% | Qsp 100% | Qsp 100% | Qsp 100% | Qsp 100% |
| Aspect de la composition | Une phase transparente | Une phase transparente | Une phase transparente | Une phase transparente | Une phase transparente |
| pH | 7,1 | 6,9 | 6,8 | 7 | 7,2 |
| Taille de bulles | 4,2 | 3,5 | 4,9 | 4,4 | 5,1 |
| Volume de mousse | 6,1 | 5,5 | 8,25 | 6,5 | 7 |
| Tenue de la mousse | 8,4 | 8,7 | 8 | 8,2 | 8 |
| Douceur | 10 | 10 | 7,5 | 8 | 7,5 |
| Rinçage | 8,5 | 9 | 8,5 | 9,5 | 9,4 |

| | | | | | |
|---|---|---|---|---|---|
| (1) Beaulight Shaa (SANYO) à 30 % de matière active, introduit en une quantité adéquate pour avoir le % de matière active indiquée dans le tableau | | | | | |
| (2) Plantacare 818 UP (COGNIS) à 53 % de matière active, introduit en une quantité adéquate pour avoir le % de matière active indiquée dans le tableau | | | | | |
| (3) Polyox resyn WSR N80 (AMERCHOL) à 100 % de matière active | | | | | |
| (4) Polyox WSR 205 (AMERCHOL) à 100 % de matière active | | | | | |
| (5) Polyox WSR N-60 K (AMERCHOL) à 100 % de matière active | | | | | |
| (6) (K-PEG 6000 LA) (KAO) à 100 % de matière active | | | | | |

La comparaison des exemples 1 à 2 selon l'invention et des exemples comparatifs montre que l'addition de composé oxyéthyléné de poids moléculaire supérieur à 500.000 à un tensioactif anionique du type Beaulight permet d'obtenir des mousses plus fines (tailles des bulles plus petites) et une grande douceur tout en conservant une bonne tenue de mousse ainsi qu'un bon rinçage, alors que l'on n'obtient pas le même résultat avec un composé oxyéthyléné ayant un poids moléculaire inférieur à 100.000.

### Exemples 3 et 4 selon l'invention et exemples comparatifs 4 à 6

| | Exemple 3 selon l'invention | Exemple 4 selon l'invention | Exemple comparatif 4 | Exemple comparatif 5 | Exemple comparatif 6 |
|---|---|---|---|---|---|
| Sodium Lauryl Glycol Carboxylate (1) | 3,9 | 7,8 | 0 | 0 | 0 |
| Monoalkyl phosphate (2) | 0 | 0 | 0 | 0 | 6,5 |
| Décyl glucoside (3) | 0 | 6,5 | 0 | 13 | 6,5 |
| Cocamidopropylbétaine (4) | 3,25 | 0 | 6,5 | 0 | 0 |
| PEG-150 pentaerythrityl tétrastéarate | 1 | 0 | 1 | 0 | 0 |
| PEG-120 méthylglucose dioleate | 1 | 0 | 1 | 0 | 0 |
| PEG-14M (5) | 1 | 1 | 1 | 1 | 1 |
| Glycérine | 10 | 0 | 10 | 0 | 0 |
| Acide citrique | 0,08 | 0,14 | 0,124 | 0,03 | 0,16 |
| Imidazolidinyl urée | 0 | 0,2 | 0 | 0,2 | 0,2 |
| phenoxyethanol / methylparaben / ethylparaben / butylparaben / iso butylparaben / propylparaben | 0 | 0,5 | 0 | 0,5 | 0,5 |
| DDM Hydantoin | 0,2 | 0,2 | 0,2 | 0 | 0 |
| Sodium Méthylparaben | 0,3 | 0,3 | 0,3 | 0 | 0 |
| Eau | Qsp100 | Qsp100 | Qsp100 | Qsp100 | Qsp100 |
| Aspect du produit | 1 phase transparente | 1 phase transparente | 2 phases | 2 phases | 2 phases |
| pH | 6,8 | 7,2 | 6,85 | 6,8 | 7,4 |

| | | | | | |
|---|---|---|---|---|---|
| (1) Beaulight Shaa (SANYO) à 30 % de matière active, introduit en une quantité adéquate pour avoir le % de matière active indiquée dans le tableau | | | | | |
| (2) MAP 20 (KAO) (100 % de matière active) | | | | | |
| (3) MYDOL10 (KAO) à 40 % de matière active, introduit en une quantité adéquate pour avoir le % de matière active indiquée dans le tableau | | | | | |
| (4) Tegobetaine E (GOLDSCHMIDT) à 31 % de matière active, introduit en une quantité adéquate pour avoir le % de matière active indiquée dans le tableau | | | | | |
| (5) Polyox WSR 205 (AMERCHOL) à 100 % de matière active | | | | | |

Ce tableau montre que l'introduction de composé oxyéthyléné de haut poids moléculaire dans des compositions moussantes provoque généralement des séparations de phases que le tensioactif présent dans la composition soit amphotère (exemple comparatif 4), non ionique (exemple comparatif 5) ou un mélande de tensioactif anionique et non ionique (exemple comparatif 6). En revanche, en présence d'un alkyl glycol carboxylate, cette séparation de phase ne se produit pas.

### Exemples 5 et 6 selon l'invention et exemples comparatifs 6 et 7

| | Exemple selon l'invention 5 | Exemple comparatif 7 | Exemple selon l'invention 6 | Exemple comparatif 8 |
|---|---|---|---|---|
| Sodium Lauryl Glycol Carboxylate (1) | 15,6 | 15,6 | 3,9 | 3,9 |
| Coco-Glucoside (2) | 0 | 0 | 3,25 | 3,25 |
| PEG-14M (3) | 1 | 0 | 1 | 0 |
| PEG-150 pentaerythrityl tétrastéarate | 0 | 0 | 1 | 1 |
| PEG-120 méthylglucose dioleate | 0 | 0 | 0,5 | 0,5 |
| Glycérine | 0 | 0 | 10 | 10 |
| Acide citrique | 0,06 | 0,03 | 0,17 | 0,19 |
| DDM Hydantoin | 0 | 0 | 0,2 | 0,2 |
| Sodium Méthylparaben | 0 | 0 | 0,3 | 0,3 |
| Imidazolidinyl urée | 0,2 | 0,2 | 0 | 0 |
| Mélange de phenoxyéthanol, methylparaben, éthylparaben, butylparaben, isobutylparaben et propylparaben | 0,5 | 0,5 | 0 | 0 |
| Eau | Qsp100 | Qsp100 | Qsp100 | Qsp100 |
| Aspect du produit | Une phase transparente | Une phase transparente | Une phase transparente | Une phase transparente |
| pH | 7,25 | 7,3 | 7,2 | 6,5 |
| Taille de bulles | 3,4 | 4,8 | 2,5 | 4,3 |
| Volume de mousse | 5,6 | 6,9 | 4,5 | 5 |
| Tenue de la mousse | 7,9 | 7,5 | 8,2 | 6,8 |
| Douceur | 9,5 | 7,5 | 9,3 | 7,5 |
| Rinçage | 8,1 | 8,5 | 8,2 | 8 |

| | | | | |
|---|---|---|---|---|
| (1) Beaulight Shaa (SANYO) à 30 % de matière active, introduit en une quantité adéquate pour avoir le % de matière active indiquée dans le tableau | | | | |
| (2) Plantacare 818 UP (COGNIS) à 53 % de matière active, introduit en une quantité adéquate pour avoir le % de matière active indiquée dans le tableau | | | | |
| (3) Polyox WSR 205 (AMERCHOL) à 100 % de matière active | | | | |

Ce tableau met en évidence les avantages des compositions selon l'invention (meilleure taille de bulle, meilleure tenue de la mousse, meilleure douceur)

### Exemple 7 selon l'invention et exemple comparatif 9

| Composition | Exemple selon l'invention 7 | Exemple comparatif 9 |
|---|---|---|
| Sodium Lauryl Glycol Carboxylate (1) | 2,2 | 2,2 |
| Coco-Glucoside (2) | 4,3 | 4,3 |
| PEG-14M (3) | 1 | 0 |
| PEG-150 pentaerythrityl tétrastéarate | 1 | 1 |
| PEG-120 méthylglucose dioleate | 0,5 | 0,5 |
| Glycérine | 10 | 10 |
| Acide citrique | 0,19 | 0,19 |
| DDM Hydantoin | 0,2 | 0,2 |
| Sodium Méthylparaben | 0,3 | 0,3 |
| Eau | Qsp100 | Qsp100 |
| Aspect du produit | Une phase transparente | Une phase transparente |
| pH | 6,8 | 6,8 |
| Taille de bulles | 3,4 | 4,5 |
| Volume de mousse | 4,9 | 5,3 |
| Tenue de la mousse | 7,9 | 7,4 |
| Rinçage | 8,4 | 8,4 |

| | | |
|---|---|---|
| (1) Beaulight Shaa (SANYO) à 30 % de matière active, introduit en une quantité adéquate pour avoir le % de matière active indiquée dans le tableau | | |
| (2) Plantacare 818 UP (COGNIS) à 53 % de matière active, introduit en une quantité adéquate pour avoir le % de matière active indiquée dans le tableau | | |
| (3) Polyox WSR 205 (AMERCHOL) à 100 % de matière active | | |

La comparaison de l'exemple selon l'invention et de l'exemple comparatif de ce tableau montre que l'addition de composés oxyéthylénés de haut poids moléculaire améliore la finesse de la mousse (petites tailles des bulles) et la tenue de la mousse tout en préservant la transparence de la composition.

### Exemple 8 selon l'invention et exemple comparatif 10

| Composition | Exemple selon l'invention 8 | Exemple comparatif 10 |
|---|---|---|
| Sodium Lauryl Glycol Carboxylate (1) | 3,9 | 3,9 |
| Cocamidopropylbétaine (2) | 3,25 | 3,25 |
| PEG-14M (3) | 1 | 0 |
| PEG-150 pentaerythrityl tétrastéarate | 1 | 1 |
| PEG-120 méthylglucose dioleate | 1 | 1 |
| Glycérine | 10 | 10 |
| Acide citrique | 0,1 1 | 0,09 |
| DDM Hydantoin | 0,2 | 0,2 |
| Sodium Méthylparaben | 0,3 | 0,3 |
| Eau | Qsp100 | Qsp100 |
| Aspect du produit | une phase transparente | une phase transparente |
| pH | 7,2 | 7,1 |
| Taille de bulles | 3,3 | 4,4 |
| Volume de mousse | 5,8 | 5,8 |
| Tenue de la mousse | 8,8 | 7,5 |
| Rinçage | 7,2 | 7,9 |

| | | |
|---|---|---|
| (1) Beaulight Shaa (SANYO) à 30 % de matière active, introduit en une quantité adéquate pour avoir le % de matière active indiquée dans le tableau | | |
| (2) Tegobetaine E (GOLDSCHMIDT) à 31 % de matière active, introduit en une quantité adéquate pour avoir le % de matière active indiquée dans le tableau | | |
| (3) Polyox WSR 205 (AMERCHOL) à 100 % de matière active | | |

Ce tableau montre que l'introduction de composé oxyéthyléné de haut poids moléculaire permet d'améliorer la qualité de la mousse (taille des bulles, tenue de la mousse, douceur) des systèmes moussants tout en préservant leur transparence.

## Revendications

1. Composition de nettoyage pour application topique, contenant, dans un milieu aqueux physiologiquement acceptable, au moins un tensioactif anionique choisi parmi les acides alkyl glycol carboxyliques et leurs sels, et au moins un composé oxyéthyléné ayant un poids moléculaire égal ou supérieur à 300.000 g/mole.

2. Composition selon la revendication 1, **caractérisée en ce que** le tensioactif anionique est présent en une quantité allant de 0,5 à 20 % en poids par rapport au poids total de la composition.

3. Composition selon la revendication 1 ou 2, **caractérisée en ce que** le tensioactif anionique a la formule (I) suivante :
R₁-CHOH-CH₂-O-CH₂-COO⁻ X⁺ (I)
dans laquelle R₁ désigne un radical alkyle saturé ou insaturé, linéaire ou ramifié, comportant de 8 à 30 atomes de carbone, et X désigne l'hydrogène ou un cation minéral ou organique.

4. Composition selon la revendication précédente, **caractérisée en ce que** le tensioactif anionique est un composé de formule (I) dans laquelle R₁ désigne un radical alkyle saturé ou insaturé, linéaire ou ramifié comportant de 8 à 18 atomes de carbone.

5. Composition selon la revendication précédente, **caractérisée en ce que** le tensioactif anionique est le lauryl glycol carboxylate de sodium, ou sa forme acide.

6. Composition selon l'une quelconque des revendications précédentes, **caractérisée en ce qu'**elle comprend en outre un ou plusieurs tensioactifs additionnels choisis parmi les tensioactifs non ioniques, amphotères et anioniques, et leurs mélanges.

7. Composition selon la revendication précédente, **caractérisée en ce que** le tensioactif additionnel est un tensioactif non ionique choisi parmi les alkylpolyglucosides.

8. Composition selon l'une quelconque des revendications précédentes, **caractérisée en ce que** la quantité totale de tensioactifs va de 2 à 50 % en poids par rapport au poids total de la composition.

9. Composition selon l'une quelconque des revendications 6 à 8, **caractérisée en ce que** le rapport en poids du tensioactif anionique choisi parmi les acides alkyl glycol carboxyliques et leurs sels, par rapport aux autres tensioactifs va de 100/0 à 10/90.

10. Composition selon l'une quelconque des revendications précédentes, **caractérisée en ce que** la quantité de composé oxyéthyléné va de 0,1 à 3 % en poids par rapport au poids total de la composition.

11. Composition selon la revendication précédente, **caractérisée en ce que** le composé oxyéthyléné est un composé de formule :
H(OCH₂CH₂) nOH
où n est un nombre entier allant de 7000 à 90.000.

12. Composition selon la revendication précédente, **caractérisée en ce que** le composé oxyéthyléné est choisi parmi PEG 14M, PEG-45M, et leurs mélanges.

13. Utilisation cosmétique d'une composition selon l'une quelconque des revendications 1 à 12, comme produit de nettoyage et/ou de démaquillage de la peau, du cuir chevelu et/ou des cheveux, et/ou comme produit de gommage et/ou produit exfoliant pour la peau.

14. Utilisation de la composition selon l'une quelconque des revendications 1 à 12, pour la préparation d'une composition destinée à traiter la peau grasse.

## Claims

1. Cleansing composition for topical application, containing, in a physiologically acceptable aqueous medium, at least one anionic surfactant chosen from alkyl glycol carboxylic acids and salts thereof, and at least one oxyethylenated compound with a molecular weight of greater than or equal to 300 000 g/mol.

2. Composition according to Claim 1, **characterized in that** the anionic surfactant is present in an amount ranging from 0.5% to 20% by weight relative to the total weight of the composition.

3. Composition according to Claim 1 or 2, **characterized in that** the anionic surfactant has the formula (I) below:
R₁-CHOH-CH₂-O-CH₂-COO⁻ X⁺ (I)
in which R₁ denotes a saturated or unsaturated, linear or branched alkyl radical containing from 8 to 30 carbon atoms and X denotes hydrogen or a mineral or organic cation.

4. Composition according to the preceding claim, characte:rized in that the anionic surfactant is a compound of formula (I) in which R₁ denotes a saturated or unsaturated, linear or branched alkyl radical containing from 8 to 18 carbon atoms.

5. Composition according to the preceding claim, **characterized in that** the anionic surfactant is sodium lauryl glycol carboxylate, or its acid form.

6. Composition according to any one of the preceding claims, **characterized in that** it also comprises one or more additional surfactants chosen from nonionic, amphoteric and anionic surfactants, and mixtures thereof.

7. Composition according to the preceding claim, **characterized in that** the additional surfactant is a nonionic surfactant chosen from alkylpolyglucosides.

8. Composition according to any one of the preceding claims, **characterized in that** the total amount of surfactants ranges from 2% to 50% by weight relative to the total weight of the composition.

9. Composition according to any one of Claims 6 to 8, **characterized in that** the weight ratio of the anionic surfactant chosen from alkyl glycol carboxylic acids and salts thereof relative to the other surfactants ranges from 100/0 to 10/90.

10. Composition according to any one of the preceding claims, **characterized in that** the amount of oxyethylenated compound ranges from 0.1% to 3% by weight relative to the total weight of the composition.

11. composition according to the preceding claim, **characterized in that** the oxyethylenated compound is a compound of formula:
H(OCH₂CH₂)ₙOH
in which n is an integer ranging from 7000 to 90 000.

12. Composition according to the preceding claim, **characterized in that** the oxyethylenated compound is chosen from PEG 14M and PEG-45M, and mixtures thereof.

13. Cosmetic use of a composition according to any one of Claims 1 to 12, as a cleansing and/or makeup-removing product for the skin, the scalp and/or the hair, and/or as a scrubbing product and/or an exfoliant product for the skin.

14. Use of the composition according to any one of Claims 1 to 12, for the preparation of a composition for treating greasy skin.

## Patentansprüche

1. Zusammensetzung für die Reinigung zur topischen Anwendung, die in einem physiologisch akzeptablen, wässrigen Medium mindestens einen anionischen grenzflächenaktiven Stoff, der unter den Alkylglycolcarbonsäuren und deren Salzen ausgewählt ist, und mindestens eine ethoxylierte Verbindung enthält, die eine Molmasse von 300.000 g/mol oder darüber aufweist.

2. Zusammensetzung nach Anspruch 1, **dadurch gekennzeichnet, dass** der anionische grenzflächenaktive Stoff in einem Mengenanteil von 0,5 bis 20 Gew.-%, bezogen auf das Gesamtgewicht der Zusammensetzung, enthalten ist.

3. Zusammensetzung nach Anspruch 1 oder 2, **dadurch gekennzeichnet, dass** der anionische grenzflächenaktive Stoff die folgende Formel (I) aufweist:
R₁-CHOH-CH₂-O-CH₂-COO⁻ X⁺ (I)
wobei R₁ eine gesättigte oder ungesättigte, lineare oder verzweigte Alkylgruppe mit 8 bis 30 Kohlenstoffatomen ist und X Wasserstoff oder ein organisches oder anorganisches Kation bedeutet.

4. Zusammensetzung nach dem vorgehenden Anspruch, **dadurch gekennzeichnet, dass** der anionische grenzflächenaktive Stoff eine Verbindung der Formel (I) ist, worin R₁ eine gesättigte oder ungesättigte, lineare oder verzweigte Alkylgruppe mit 8 bis 18 Kohlenstoffatomen bedeutet.

5. Zusammensetzung nach dem vorgehenden Anspruch, **dadurch gekennzeichnet, dass** der anionische grenzflächenaktive Stoff das Natriumlaurylglycolcarboxylat oder seine Säureform ist.

6. Zusammensetzung nach einem der vorhergehenden Ansprüche, **dadurch gekennzeichnet, dass** sie ferner einen oder mehrere ergänzende grenzflächenaktive Stoffe enthält, die unter den nichtionischen, amphoteren und anionischen grenzflächenaktiven Stoffen und deren Gemischen ausgewählt ist.

7. Zusammensetzung nach dem vorgehenden Anspruch, **dadurch gekennzeichnet, dass** der ergänzende grenzflächenaktive Stoff ein nichtionischer grenzflächenaktiver Stoff ist, der unter den Alkylpolyglucosiden ausgewählt ist.

8. Zusammensetzung nach einem der vorhergehenden Ansprüche, **dadurch gekennzeichnet, dass** die Gesamtmenge der grenzflächenaktiven Stoffe im Bereich von 2 bis 50 Gew.-%, bezogen auf das Gesamtgewicht der Zusammensetzung, liegt.

9. Zusammensetzung nach einem der Ansprüche 6 bis 8, **dadurch gekennzeichnet, dass** das Gewichtsverhältnis des anionischen grenzflächenaktiven Stoffes, der unter den Alkylglycolcarbonsäuren und deren Salzen ausgewählt ist, und der anderen grenzflächenaktiven Stoffe im Bereich von 100/0 bis 10/90 liegt.

10. Zusammensetzung nach einem der vorhergehenden Ansprüche, **dadurch gekennzeichnet, dass** der Mengenanteil der ethoxylierten Verbindung im Bereich von 0,1 bis 3 Gew.-%, bezogen auf das Gesamtgewicht der Zusammensetzung, liegt.

11. Zusammensetzung nach dem vorgehenden Anspruch, **dadurch gekennzeichnet, dass** die ethoxylierte Verbindung eine Verbindung der folgenden Formel ist:
H(OCH₂CH₂)ₙOH
wobei n eine ganze Zahl von 7.000 bis 90.000 ist.

12. Zusammensetzung nach dem vorgehenden Anspruch, **dadurch gekennzeichnet, dass** die ethoxylierte Verbindung unter PEG 14M, PEG-45M und deren Gemischen ausgewählt ist.

13. Kosmetische Verwendung einer Zusammensetzung nach einem der Ansprüche 1 bis 12 als Produkt zur Reinigung und/oder zum Abschminken der Haut, der Kopfhaut und/oder der Haare und/oder als Peeling-Produkt und/oder als exfoliatives Produkt für die Haut.

14. Verwendung der Zusammensetzung nach einem der Ansprüche 1 bis 12 für die Herstellung einer Zusammensetzung, die für die Behandlung fettiger Haut vorgesehen ist.
